(19) Europäisches Patentamt / European Patent Office / Office européen des brevets

(11) **EP 3 270 871 B1**

(12) **EUROPEAN PATENT SPECIFICATION**

(45) Date of publication and mention
of the grant of the patent:
**03.07.2019 Bulletin 2019/27**

(21) Application number: **16713766.0**

(22) Date of filing: **16.03.2016**

(51) Int Cl.:
*C08K 7/18* (2006.01)  *A61K 8/25* (2006.01)
*A61Q 1/02* (2006.01)  *A61K 8/02* (2006.01)
*C08K 3/34* (2006.01)

(86) International application number:
**PCT/EP2016/055730**

(87) International publication number:
**WO 2016/146708 (22.09.2016 Gazette 2016/38)**

(54) **COSMETIC COMPOSITIONS COMPRISING A TALC PARTICULATE**

EINES TEILCHENFÖRMIGEN TALKS ENTHALTENDE KOSMETISCHE ZUSAMMENSETZUNG

COMPOSITION COSMETIQUE COMPRENANT UN TALC SOUS FORME PARTICULAIRE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **18.03.2015 EP 15290077
20.04.2015 EP 15164298
11.01.2016 EP 16305020**

(43) Date of publication of application:
**24.01.2018 Bulletin 2018/04**

(73) Proprietor: **IMERTECH SAS
75015 Paris (FR)**

(72) Inventors:
• **MELI, Gilles
31410 Le Fauga (FR)**
• **PAGIS, Laure
31500 Toulouse (FR)**

(74) Representative: **Haseltine Lake Kempner LLP
Redcliff Quay
120 Redcliff Street
Bristol BS1 6HU (GB)**

(56) References cited:
**EP-A1- 2 410 008    WO-A1-97/04737
US-A- 5 849 333    US-A1- 2006 159 643**

• **Imerys talc: "Talc - Italian - BC(Cosmetic Grade)",
Brenntag-Specialties , September 2012
(2012-09), XP002742808, Retrieved from the
Internet:
URL:http://www.brenntagspecialties.com/en/
downloads/Products/Personal_care/Talcs/Com
pare_Talc_Cos_Italian.pdf [retrieved on
2015-07-27]**
• **MITCHELL L. SCHLOSSMAN, B.S., and ADRIAN
J. FELDMAN, B.S: "The Evaluation of Chemical
Binders and Their Effect on Pressed Powders",
J.Soc.Cosmet.Chem., vol. 24 23 May 1973
(1973-05-23), pages 357-362, XP002742809,
Retrieved from the Internet:
URL:http://journal.scconline.org/pdf/cc197
3/cc024n06/p00357-p00362.pdf [retrieved on
2015-07-27]**
• **Anonymous: "Physicians Formula-Multi-Colored
Pressed Powder", ULTA Salon, Cosmetics &
Fragrance, , 24 March 2012 (2012-03-24),
XP002742810, Retrieved from the Internet:
URL:http://wayback.archive.org/web/2012032
4150548/http://www.ulta.com/ulta/browse/pr
oductDetail.jsp?productId=1378#details
[retrieved on 2015-07-27]**
• **IMERYS TALC: "Product Data Sheet-Extra 5/0
DEC", INTERNET CITATION, 2012, page 1,
XP002743911, Retrieved from the Internet:
URL:http://www.brenntagspecialties.com/en/
downloads/Products/Personal_care/Talcs/Ext
ra_50_DEC_TDS_190.pdf [retrieved on
2015-09-03]**

**Description**

**TECHNICAL FIELD**

The present invention is directed to cosmetic compositions comprising a talc particulate which is used in the cosmetic composition as a colour booster.

**BACKGROUND OF THE INVENTION**

Inorganic particulate materials, such as talc particulate, are commonly used as fillers in cosmetic products, such as pressed powders. There is an ongoing need to develop new cosmetic products having enhanced optical and physical properties.

**SUMMARY OF THE INVENTON**

According to a first aspect, the present invention is directed to a cosmetic composition comprising colourant and a talc particulate having a $d_{50laser}$ of at least 9.0 $\mu$m, a lamellarity index of at least 1.0, and wherein the talc particulate comprises less than 20 % aluminium, based on the weight of the talc particulate. In certain embodiments, the talc particulate has a $d_{50laser}$ of at least 10.0 $\mu$m, a lamellarity index of at least 1.0, and comprises less than 10 % aluminium, based on the weight of the talc particulate.

According to a second aspect, the present invention is directed to a cosmetic composition for application to human skin, said cosmetic composition comprising colourant and a colour boosting amount of the talc particulate as defined above, wherein the talc particulate boosts the colour of the cosmetic composition (i) such that a lower amount of the cosmetic composition may be applied to any given surface area of the skin without loss of colour, and/or (ii) such that the colour density of the cosmetic composition is boosted compared to the cosmetic composition absent the talc particulate, and/or (iii) a lower amount of colourant may be used without loss of colour density compared to the cosmetic composition absent the talc particulate.

According to a third aspect, the present invention is directed to a cosmetic method for modifying the visual appearance of a facial feature, comprising applying, to the facial feature, a cosmetic composition comprising colourant and a colour boosting amount of the talc particulate as defined above.

According to a fourth aspect, the present invention is directed to the use as a colour booster in a cosmetic composition of the talc particulate as defined above.

According to a fifth aspect, the present invention is directed to the use as a colour booster in a pressed powder cosmetic of a talc particulate, wherein the talc particulate has $d_{50laser}$ of at least 9.0 $\mu$m, and a lamellarity index of at least 1.0., wherein the talc particulate comprises less than 20 % aluminium, based on the weight of the talc particulate In certain embodiments, the talc particulate has a $d_{50laser}$ of at least 10.0 $\mu$m, a lamellarity index of at least 1.0, and comprises less than 10 % aluminium, based on the weight of the talc particulate.

**BRIEF DESCRIPTION OF THE DRAWINGS**

Fig. 1 (not part of the invention as claimed) is a graph showing the fracture resistance of a series of pressed tablets prepared in accordance with Example 2.

Fig. 2 (not part of the invention as claimed) comprises photographs of a series of cosmetic compacts prepared in accordance with Example 3.

**DETAILED DESCRIPTION OF THE INVENTION**

It has surprisingly been found that certain inorganic particulate materials, particularly talc particulates, may be incorporated in cosmetic preparations to enhance one or more optical and physical properties thereof, in certain embodiments providing an unexpected balance of properties. In particular, incorporation of the inorganic particulate materials described herein, such as a talc particulate, may boost the colour (i.e., increase the colour density) of the cosmetic. The various properties described above may be determined in accordance with the methods described herein or any other suitable panel or instrumental test.

As used herein, the term "cosmetic composition" means a composition intended to be applied to the human body for beautifying, promoting attractiveness, or altering the appearance without affecting the body's structure or functions. In certain embodiments, the cosmetic composition is a decorative cosmetic.

In certain embodiments, the cosmetic composition is a powder (e.g., pressed or loose), a liquid, a gel, a cream (e.g., a cream emulsion), a dispersion or an anhydrous stick. In certain embodiments, the cosmetic composition is a powder, for example, a pressed powder such as, but not limited to, a powdered cosmetic compact. In certain embodiments, the cosmetic composition is a makeup, for example, a face makeup including, but not limited to, primer, concealer, foundation, blush (also known as rouge or blusher), bronzer, eye shadow, mascara, lipstick, contour powder, face powder (often used to set a foundation), highlighter, eyeliner, or eyebrow applicator (e.g., pencil). Loose powders include body powder, for example, baby powder. In certain embodiments, the make-up is a luminous make-up.

As used herein, the term "lamellarity index" is defined by the following ratio:

$$\frac{d_{50laser} - d_{50sedi}}{d_{50sedi}}$$

in which "$d_{50laser}$" is the value of the mean particle size ($d_{50}$) obtained by a particle size measurement by wet Malvern laser scattering (standard ISO 13320-1) and "$d_{50sedi}$" is the value of the median diameter obtained by sedimentation using a sedigraph (standard ISO 13317-3), as described below. Reference may be made to the article by G. Baudet and J. P. Rona, Ind. Min. Mines et Carr. Les techn. June, July 1990, pp 55-61, which shows that this index is correlated to the mean ratio of the largest dimension of the particle to its smallest dimension.

In the sedimentation technique referred to above, particle size properties referred to herein for the talc particulate materials are as measured in a well known manner by sedimentation of the particulate material in a fully dispersed condition in an aqueous medium using a Sedigraph 5100 machine as supplied by Micromeritics Instruments Corporation, Norcross, Georgia, USA (www.micromeritics.com), referred to herein as a "Micromeritics Sedigraph 5100 unit", and based on application of Stokes' Law. Such a machine provides measurements and a plot of the cumulative percentage by weight of particles having a size, referred to in the art as the equivalent spherical diameter' (e.s.d), less than given e.s.d values. The mean particle size $d_{50}$ is the value determined in this way of the particle e.s.d at which there are 50% by weight of the particles which have an equivalent spherical diameter less than that $d_{50}$ value. The $d_{95}$ value is the value at which 95% by weight of the particles have an esd less than that $d_{95}$ value. Particle size properties may be determined in accordance with ISO 13317-3, or any method equivalent thereto.

In the Malvern laser light scattering technique referred to above, the size of particles in powders, suspensions and emulsions may be measured using the diffraction of a laser beam, based on an application of Mie theory. Such a machine, for example a Malvern Mastersizer S (as supplied by Malvern Instruments) provides measurements and a plot of the cumulative percentage by volume of particles having a size, referred to in the art as the 'equivalent spherical diameter' (e.s.d), less than given e.s.d values. The mean particle size $d_{50}$ is the value determined in this way of the particle e.s.d at which there are 50% by weight of the particles which have an equivalent spherical diameter less than that $d_{50}$ value. Particle size properties may be determined in accordance with ISO ISO 13320-1, or any method equivalent thereto. For the avoidance of doubt, the measurement of particle size using laser light scattering is not an equivalent method to the sedimentation method referred to above.

As used herein, "specific surface area (BET)" means the area of the surface of the particles of the talc particulate with respect to unit mass, determined according to the BET method by the quantity of nitrogen adsorbed on the surface of said particles so to as to form a monomolecular layer completely covering said surface (measurement according to the BET method, AFNOR standard X11-621 and 622 or ISO 9277). In certain embodiments, specific surface area is determined in accordance with ISO 9277, or any method equivalent thereto.

*Inorganic particulate material*

The inorganic particulate material is talc as defined in the claims. The talc particulate has a $d_{50laser}$ of at least 9.0 $\mu$m. In such embodiments, the talc particulate may have a lamellarity index of at least 1.0, preferably at least 3.0 or at least 4.0.

In certain embodiments, the talc particulate has a $d_{50laser}$ of at least 10.0 $\mu$m and a lamellarity index of at least 1.0.

In certain embodiments, the talc particulate has a lamellarity index of from 6.0 to 8.0, for example, from 6.5 to 7.5, a $d_{50laser}$ of from 20.0 $\mu$m to 25.0 $\mu$m, and optionally a $d_{50sedi}$ of from 2.5 $\mu$m to 3.5 $\mu$m.

In certain embodiments, the talc particulate has a lamellarity index of from 1.0 to 3.0, for example, from 1.0 to 2.5, or from 1.25 to 2.0, a $d_{50laser}$ of from 30.0 $\mu$m to about 40.0 $\mu$m, and optionally a $d_{50sedi}$ of from 10.0 $\mu$m to 15.0 $\mu$m.

In certain embodiments, the talc particulate has a $d_{95laser}$ of less than 100 $\mu$m, for example, less than 90 $\mu$m, or less than 80 $\mu$m, or less than 75 $\mu$m, or less than 70 $\mu$m, or less than 65 $\mu$m, or less than 60 $\mu$m, or less than 55 $\mu$m, or less than 50 $\mu$m.

The talc particulate additionally has an aluminium content of less than 20 %, based on the total weight of the talc particulate. The aluminium content is calculated as $Al_2O_3$ content, as may be determined by X-ray Fluorescence Spectroscopy (XFS).

In certain embodiments, the talc is derived from a synthetic talc, also known as talcose. In certain embodiments, the talc is an Italian talc, i.e., derived from a natural talc resource based in Italy. In certain embodiments, the talc is a French talc, i.e., derived from a natural talc resource based in France.

In certain embodiments, the talc particulate has a specific surface area (BET) equal to or greater than about 3 $m^2/g$, for example, equal to or greater than about 8 $m^2/g$, equal to or greater than about 8 $m^2/g$, or equal to or greater than about 15 $m^2/g$, or equal to or greater than about 20 $m^2/g$. In certain embodiments, talc particulate has a specific surface area (BET) of from about 5 $m^2/g$ to about 30 $m^2/g$, for example, from about 10 $m^2/g$ to about 25 $m^2/g$, or from about 15 $m^2/g$ to about 25 $m^2/g$, or from about 20 $m^2/g$ to about 25 $m^2/g$. In certain embodiments, talc particulate has a specific surface area (BET) of from about 3 $m^2/g$ to about 10 $m^2/g$, for example, from about 3 $m^2/g$ to about 8 $m^2/g$, or from about 3 $m^2/g$ to about 7 $m^2/g$, or from about 4 $m^2/g$ to about 6 $m^2/g$.

In certain embodiments, the talc particulate has:

(i) a specific surface area (BET) of least about 10.0 $m^2/g$ for a $d_{50sedi}$ between 2.0 - 8.0 $\mu$m;
(ii) a specific surface area (BET) of least about 5.0 $m^2/g$ for a $d_{50sedi}$ between 8.0 - 15.0 $\mu$m;
(iii) a lamellarity index of at least about 3.0 for a $d_{50sedi}$ between 2.0 - 8.0 $\mu$m; and/or
(iv) a lamellarity index of at least about 1.0 for a $d_{50sedi}$ between 8.0-15.0 $\mu$m.

In certain embodiments, the talc particulate may be characterised in terms oil absorption. A higher relative oil absorption may enhance one or properties of the cosmetic composition such as, for example, sebum absorption. In certain embodiments, the talc particulate has an oil absorption of at least about 50 %, for example, at least about 60 %, or at least about 70 %. Oil absorption (i.e., amount of oil absorbed per amount of particulate, e.g., ml or g of oil per 100 g of particulate) may be determined by any suitable method, for example, ASTM D1483.

In certain embodiments, the talc particulate is uncoated.

As mentioned above, it has surprisingly been found that talc particulate may be used to favourably enhance one or more optical and/or physical properties of a cosmetic composition comprising colourant. Particularly, the talc particulate is used as a colour booster to boost the colour of the cosmetic composition. By "boost the colour" or "colour booster" is meant that the colour density of the cosmetic composition is enhanced by incorporation of the talc particulate, as may be determined by spectrocolorimetery in accordance with ISO 12647. The colour density of the cosmetic composition is boosted compared to the cosmetic composition absent the talc particulate, for example, absent the talc particulate having the characteristics (e.g., the particle size distribution) described herein.

Thus, when applied to human skin, the colour of the cosmetic composition comprising colourant and the colour boosting amount of the talc particulate is boosted, meaning that a lower amount of the cosmetic composition may be applied to any given surface area of the skin without loss of colour and/or a lower amount of colourant may be employed without loss of colour density. Being able to use less cosmetic without loss of colour and/or inclusion of less pigment is economically and environmentally beneficial.

As such, in certain embodiments, there is provided a cosmetic method for modifying the visual appearance of a facial feature, comprising applying, to the facial feature, a cosmetic composition comprising colourant and a colour boosting amount of the talc particulate. The presence of the colour boosting amount of the talc particulate boosts the colour such that (i) a lower amount of the cosmetic composition may be applied to any given surface area of the facial feature without loss of colour and/or (ii) a lower amount of colourant may be used without loss of colour density. In certain embodiments, the assessment is made relative to a comparable cosmetic composition comprising no talc particulate or comprising the same amount of a talc particulate having a $d_{50laser}$ of less than 9.0 $\mu$m and/or a lamellarity index of less than 1.0. In certain embodiments, the facial feature is one or more of skin, lips, eyebrow or eyelash.

In certain embodiments, the talc particulate is used as a colour booster in a pressed powder cosmetic compact. The wet processing method comprises preparing an aqueous slurry of the talc particulate and any other components of the pressed powder cosmetic compact, for example, colourant (such as mineral pigment) and binder, and then de-watering the slurry, e.g., by drying at a suitably elevated temperature (e.g., above about 70 °C), and then pressing to form a pressed powder cosmetic compact.

In certain embodiments, the cosmetic composition comprises from 1 % by weight to 95 % by weight of talc particulate (i.e., based on the total weight of the cosmetic composition) for example, from 20 % to 90 % by weight, or from 30 % to 90 % by weight, or from 40 % to 90 % by weight, or from 50 % to 90 % by weight, or from 60 % to 90 % by weight, or from 65 % to 85 % by weight, or from 70 % to 85 % by weight, or from 75 % to 85 % by weight, or up to 95 % by weight talc particulate, or up to 90 % by weight talc particulate, or up to 85 % by weight talc particulate, or up to 80 % by weight talc particulate.

In certain embodiments, the cosmetic composition comprises a combination of a higher grade talc particulate and a lower grade talc particulate. For example, the higher grade talc particulate may have a $d_{50laser}$ of at least 10.0 $\mu$m, a lamellarity index of at least 1.0 and an aluminium content of less than 10 %, and further embodiments thereof in accordance with the talc particulate described above, and the lower grade talc particulate may have a coarser particle size distribution and/or a lamellarity index of less than 1.0 (or less than 0.75, or less than 0.5, or less than 0.25) and/or an aluminium content which is higher than the higher grade talc particulate, for example, an aluminium content of equal to or greater than 10 %, or equal to or greater than 7.5 %, or equal to or greater than 5 %. A higher grade talc particulate may require a higher degree of engineering and, thus, may be more expensive to produce compared to a lower grade talc particulate.

The talc particulate may be obtained from a natural source by grinding. Natural talc particulate is typically obtained by crushing and then grinding a mineral source of talc, which may be followed by a particle size classification step, in order to obtain a product having a desired particle size distribution. The particulate solid material may be ground autogenously, i.e. by attrition between the particles of the solid material themselves, or, alternatively, in the presence of a particulate grinding medium comprising particles of a different material from the talc particulate to be ground. These processes may be carried out with or without the presence of a dispersant and biocides, which may be added at any stage of the process.

The talc particulate may be prepared using techniques well known to a person of skill in the art, for example, techniques selected from comminution (e.g., crushing, grinding, milling), classification (e.g., hydrodynamic selection, screening and/or sieving) and drying.

In certain embodiments, the talc particulate is prepared by a process according to that described in US-A-6348536. More particularly, the talc particulate may be prepared by a process comprising:

(a) talc with a predetermined initial particle size is suspended in a liquid,
(b) the suspension is subjected to a delamination operation adapted so as to produce a separation of the leaves of the particles and so as to obtain a particle size less than the initial particle size,
(c) optionally subjecting the suspension to a selection as to eliminate particles with a size greater than a predetermined size,
(d) drying the suspension, and
(e) optionally treating the particles so as to limit the creation of strong bonds between them.

The starting talc is typically chosen having an initial particle size which is greater than the desired particle size. In certain embodiments, the starting talc is suspended in water in the presence of a dispersing agent such that the weight of dry matter based on the total weight of the suspension is from about 10 % to about 60 %. The suspension is typically homogenous. The grinding operation during delamination is, in certain embodiments, carried out as to obtain a $d_{50laser}$ of from 10 $\mu$m to 50 $\mu$m. The selection step may comprise hydrodynamic selection, which may be carried out in a turbine selector or in a hydrocyclone or in a centrifuge with an endless extraction screw. The suspension is advantageously dried in such a way as to reach a residual liquid level below 1 %.

In certain embodiments, the talc particulate is prepared by a process comprising:

(a) delaminating a liquid suspension of a relatively coarse talc particulate having an initial particle size with a $d_{50laser}$ which is greater than a desired $d_{50laser}$ (e.g., greater than a desired $d_{50laser}$ of at least 9.0 $\mu$m, or from 10 $\mu$m to 50 um, or from 10 $\mu$m to 35 $\mu$m), to obtain a talc particulate having a particle size less than the initial particle size;
(b) at least partially drying the suspension thereby obtaining a talc particulate having the desired $d_{50laser}$ and optionally a desired lamellarity index.

In certain embodiments, the desired $d_{50laser}$ is at least 10 $\mu$m, and the desired lamellarity index is at least 1.0.

In certain embodiments, the inorganic particulate, e.g. the talc particulate is not chemically treated during processing to obtain the desired particle size and lamellarity.

In certain embodiments, the cosmetic composition comprises colourant and/or binder and/or cosmetically acceptable base in addition to the inorganic particulate material. In certain embodiments, the binder, when present, may be a constituent of the cosmetically acceptable base. In certain embodiments, the cosmetic composition comprises colourant and binder, in addition to the talc particulate.

The colourant (i.e., a component which imparts colour) may be an organic colourant and/or an inorganic colourant. Colourants for cosmetics are many and various. A list of colorant agents permitted for use in cosmetic products is provided in Annex IV to the Cosmetics Directive 76/768/EEC. Organic colourants include dyes and the like. Examples of organic colourants include species characterized in one of the following groups: indigoid, xanthenes, azo, nitro, triphentlmehtnae, quinoline and anthraquinone. Inorganic colourants include pigments, such as mineral pigments. In certain embodiments, the colourant is a mineral pigments, for example, one or more of zinc oxide, titanium dioxide, iron oxide (black, red, orange, yellow and/or brown), tin oxide, chrome oxide, ultramarine (blue, pink and/or violet), manganese violet (ammonium manganese (III) pyrophosphate), Prussian blue (ferric ferrocynanide) and mica. In certain embodiments, the colourant is a pigment. In certain embodiments, the colourant is a mineral pigment. The colourant, for example, the mineral pigment or combinations thereof, may be selected depending on the desired colour for the cosmetic. In certain embodiments, the colourant may be nacre or a derivative thereof, providing a desirable pearlescence (also known as luster) and/or brilliance. In such embodiments, the incorporation of the inorganic particulate, for example, talc particulate, may function as a nacre-booster, serving either to enhance the pearlescence and/or brilliance of the cosmetic composition in the presence of nacre, or enable a lower amount of nacre to be used without loss of pearlescence and/or brilliance.

The colourant may constitute up to about 40 % by be weight of the cosmetic composition, for example, up to about 35 % by weight, or up to about 30 % by weight, or up to about 35 % by weight, or up to about 20 % by weight of the cosmetic composition, for example, from about 0.001 % to about 20 % by weight, or from about 0.01 % to about 20 % by weight, or from about 0.1 % to about 20 % by weight, or from about 1.0% to about 20 % by weight, or from about 1.0 % to about 15 % by weight, or from about 2.0 % to about 15 % by weight, or from about 5.0 % to about 15 % by weight, or from about 7.5 % by weight to about 12.5 % by weight of the cosmetic composition.

In certain embodiments, the cosmetic composition is an eye-shadow, for example, a highly coloured eye-shadow. In certain embodiments, the cosmetic composition is an eyeshadow and comprises at least about 30 % by weight of talc particulate and at least about 10 % by weight of colourant, for example, at least about 35 % by weight talc particulate and at least about 20 % by weight colourant, or at least about 40 % by weight talc particulate and at least about 25 % by weight colourant. In such embodiments, the cosmetic composition, i.e., eye-shadow may comprises no more than about 50 % by weight talc particulate, for example, no more than about 45 % by weight talc particulate, and no more than about 40 % by weight colourant, for example, no more than about 35 % by weight colourant, or no more than about 30 % by weight colourant.

In certain embodiments, the cosmetic composition is a blush and comprises from about 60 % by weight to about 90 % by weight talc particulate, for example, from about 70 % by weight to about 85 % by weight talc particulate, or from about 75 % by weight to about 85 % by weight talc particulate, or from about 75 % by weight to about 80 % by weight talc particulate.

In certain embodiments, the cosmetic composition is a foundation and comprises from at least about 70 % by weight talc particulate, for example, at least about 80 % by weight talc particulate, or at least about 90 % by weight talc particulate. In such embodiments, the cosmetic composition, for example, foundation, may comprise from about 0.1 % by weight to about 10 % by weight colourant, for example, from about 0.5 % by weight to about 7 % by weight colourant, or from about 1.0 % by weight to about 5 % by weight colourant, or at least about 2 % by weight colourant, or at least about 3 % by weight colourant.

When present, the cosmetically acceptable base may be any base suitable for the intended purpose. In certain embodiments, the base is an oil and/or wax containing material. The base and, thus, the cosmetic composition, may comprise other components such as humectants, preservative, emollient, fragrance and antioxidant.

The binder, when present, may be a solid or liquid binder. In certain embodiments, the binder is a liquid binder, for example, on oil-based binder. In certain embodiments, the liquid binder is a fatty acid or ester or salt thereof, or a combination of fatty acids and/or ester and/or salts thereof. In certain embodiments, the fatty acid or ester or salt thereof, or combinations thereof, is derived from vegetable oil, for example, coconut oil, palm oil, palm kernel oil soybean oil, corn oil, rapeseed oil, and the like. In certain embodiments, the binder is cocoate ester, for example, isoamyl cocoate. Other binders include silicone, sorbitan, calcium phosphate, bentonite, natural guns, rosin and cellulose.

Suitable binder materials include polyhydric alcohol, hyaluronic acid and its salts, an amino acid and its salts, chondroitin sulfuric acid and its salts, lactic acid and its salts, pyroglutamic acid and its salts, uric acid and its salts, and mixtures thereof. Polyhydric alcohols include glycerin, diglycerin, triglycerin, ethylene glycol, diethylene glycol, propylene glycol, dipropylene glycol, hexylene glycol, 1,3-butylene glycol, 1,4-butylene glycol, glucose, maltose, sucrose, xylitose, sorbitol, maltitol, malbit, panthenol, hyaluronic acid and its salts, and mixtures thereof.

Further non-limiting examples of suitable binder materials are polyglycerin fatty acid esters, propylene glycol fatty acid esters, glycerin fatty acid esters, sorbitan fatty acid esters, sugar fatty acid esters, polyoxyethylene sorbitan fatty acid esters, polyoxyethylene sorbit fatty acid esters, polyethylene glycol fatty acid esters, polyoxyethylene castor oils, polyoxyethylene hardened castor oils, polyoxyethylene alkyl ethers, polyoxyethylene phytosterols, polyoxyethylene polyoxypropylene alkyl ethers, polyoxyethylene alkyl phenyl ethers, polyoxyethylene lanolins, polyoxyethylene lanolin alcohols, polyoxyethylene beeswax derivatives, polyoxyethylene fatty acid amides, and polyether silicone derivatives.

The fatty acids making the esters above can be saturated or unsaturated, straight or branched, and include those of natural origin having about 16-18 carbons. Non-limiting examples include triglyceryl beeswax, triglyceryl cetyl ether, tetraglyceryl cocoate, triglyceryl decyltetradecanol, diglyceryl diisostearate, triglyceryl diisostearate, decaglyceryl diisostearate, diglyceryl dioleate, triglyceryl dioleate, hexaglyceryl dioleate, decaglyceryl dioleate, triglyceryl distearate, hexaglyceryl distearate, decaglyceryl distearate, decaglyceryl trioleate, decaglyceryl heptaoleate, decaglyceryl heptastearate, hexaglyceryl hexaoleate, diglyceryl isostearate, tetraglyceryl isostearate, hexaglyceryl monoisostearate, diglyceryl lanolin alcohol ether, tetraglyceryl lauryl ether, diglyceryl oleate, triglyceryl oleate, tetraglyceryl oleate, hexaglyceryl oleate, diglyceryl oleyl ether, tetraglyceryl oleyl ether, diglyceryl sesquiisostearate, and diglyceryl sesquioleate and mixtures thereof.

Also suitable as binder materials are liquid paraffin, squalane, liquid petrolatum, mineral oil, and liquid polybutene.

Also suitable are natural oils which are typically a mixture of saturated and unsaturated fatty acid. Non-limiting examples of natural oil derived from plants include almond oil, olive oil, sesame oil, safflower oil, avocado oil, cottonseed oil, jojoba oil, castor bean oil, castor oil, rapeseed oil, soybean oil, palm kernel oil, coconut oil, hydrogenated vegetable oil, and cocoa butter. Non-limiting examples of natural oil derived from animal sources include mink oil and egg yolk oil.

Non-limiting examples of fatty alcohol which may be employed as binder are isostearyl alcohol, lanolin alcohol, oleyl alcohol, hexadecyl alcohol, octyldodecanol alcohol, linoleyl alcohol, linolenyl alcohol, and arachidyl alcohol.

Fatty acid can be natural or synthetic, saturated, unsaturated, linear, or branched. Non-limiting examples of fatty acid are adipic, caprylic, capric, isostearic, linoleic, ricinoleic, oleic, elaidic and erucic acid.

Non-limiting examples of fatty acid ester are cetyl ricinoleate, cetyl oleate, cetyl octanoate, cetyl acetate, glyceryl trioctanoate, isopropyl lanolate, isopropyl linoleate, isopropyl myristate, isopropyl palmitate, isopropyl oleate, isopropyl stearate, ethyl lactate, ethyl glutamate, ethyl laurate, ethyl linoleate, ethyl methacrylate, ethyl myristate, ethyl palmitate, diisopropyl adipate, octyl dodecyl myristate, octyl palmitate, octyl isopelargonate, octyl dodecyl lactate, tridecyl isononanoate, isotridecyl isononanoate, hexadecyl stearate, oleyl oleate, isononyl isononanoate, isostearyl myristate, dipentaerythrytol ester, neopentyl glycol dioctanoate, and di(capryl/capric acid) propylene glycol and mixtures thereof. Other suitable esters include triglycerides such as caprylic triglycerides, capric triglyceride, isostearic triglyceride, adipic triglyceride and cholesterol derivatives such as cholesteryl oleate.

Non-volatile, straight, and branched silicone oil such as dimethicone and phenyl dimethicone is also useful.

The binder may constitute up to about 80 % by weight of the cosmetic composition, for example, from about 1 % to about 70 % by weight, or from about 1 % to about 69 % by weight, or from about 1 % to about 50% by weight, or from about 1 % to about 40 % by weight, or from about 1 to about 30 % by weight, or from about 1 to about 20 % by weight, or from about 2.5 % to about 15 % by weight, or from about 5 % to about 15 % by weight, or from about 7.5 % to about 12.5 % by weight of the cosmetic composition.

In certain embodiments, the cosmetic composition comprises up to about 95 % by weight talc particulate, from about 1.0 % to about 20 % by weight colourant (e.g., mineral pigment) and from about 1.0 % to about 20 % by weight binder. In such embodiments, the cosmetic composition may further comprise suitable amounts of one or more of humectants, preservative, emollient, fragrance and antioxidant, for example, up to about 10 % by weight so such components, based on the total weight of the composition, or up to about 5 % by weight of such components, or from about 0.001 % to about 2.5 % by weight of such components. In certain embodiments, the cosmetic composition is free of components other than the talc particulate, colourant and binder. The cosmetic composition may be prepared by any suitable or conventional method well known to those skilled in the art. Such methods generally comprise combining the components of the cosmetic in a liquid, slurry or solid form, mixing the components, optionally milling the mixture of components, and then forming the cosmetic composition therefrom. The components may be brought together in a blender or other mixing apparatus under conditions of suitably low shear so as to preserve the inherent properties of the particulate material. Forming may comprise drying and/or pressing, depending on the nature of the method of manufacture and the final form of the cosmetic.

In certain embodiments, the inorganic particulate, for example, talc particulate is surface treated. The surface treatment may serve to modify a property of the talc particulate and/or the cosmetic composition in which it is incorporated. In certain embodiments, the surface treatment enhances the spreadability of the cosmetic composition. In certain embodiments, the surface treatment enhances the adhesion, water repellence, sebum absorption and/or surface smoothness of the cosmetic composition, for example, when applied to human skin.

In certain embodiments, the talc particulate is surface treated to increase the hydrophobicity or lipophilicity of the talc particulate.

In certain embodiments, the talc particulate is surface treated to increase the hydrophilicity or lipophobicity of the talc particulate.

In certain embodiments, the surface treatment is an organosilane, a organophosphour, an organosulfur, or a mixture thereof.

In certain embodiments, the surface treatment agent is a hydrocarbyl phosphonic acid, for example, an alkyl phosphonic acid such as, for example, a $C_6$-$C_{24}$ phosphonic acid , for example, n-octadecylphosphonic acid. In certain embodiments, the surface treatment agent is a halo-alkyl phosphonic acid such as, for example, a fluoro-alkyl phosphonic acid such as, for example, nonafluoropentadecyl phosphonic acid.

In certain embodiments, the surface treatment is selected from one or more methicone, diemthicone, triethoxysilane, lauroyl lysine, $C_{9-15}$ flouroalcohol phosphates, magnesium myristate, triethoxy caprylsilane, polyhdyroxystearic acid and perflourooctyl triethoxysilane,

In certain embodiments, the surface treatment is or comprises microcrystalline cellulose.

In certain embodiments, the surface treatment is or comprises polyacrylate.

In certain embodiments, the surface treatment is or comprises a polyoxyalkylene (POA), for example, polyalkylene glycol (PAG) or polyalkylene oxide (PAO). As used herein, the term 'polyalkylene glycol' means a POA having a number average molecular mass below 20,000 g/mol, and the term 'polyalkylene oxide' means a POA having a number average molecular mass above 20,000 g/mol. In certain embodiments, the surface treatment comprises or is a polyalkylene glycol having a number average molecular mass of from about 100 to about 15,000 g/mo, for example, from about 200 to about 10,000 g/mol, or from about 500 to about 9000 g/mol, or from about 1000 to about 9000 g/mol, or from about 2000 to about 900 g/mol, or from about 4000 to about 9000 g/mol, or from about 6000 to about 9000 g/mol, or from about 6000 to about 8500 g/mol.

In certain embodiments, the surface treatment is or comprises a polyalkylene oxide selected from one or more of paraformaldehyde (polymethylene oxide), polytetramethylene glycol, polytetramehtylene ether glycol, polyethylene oxide, polypropylene oxide, polybutylene oxide, and combinations thereof.

In certain embodiments, the surface treatment is or comprises a polyethylene glycol. In certain embodiments, the surface treatment comprises or is a mixture of polyethylene glycol and polypropylene glycol (PPG).

In certain embodiments, the surface treatment is or comprises a polyether modified polysiloxane. The polyether modified polysiloxane may be derived from a linear polysiloxane. In certain embodiments, the polyether modified polysiloxane is derived from poly(dimethylsiloxane), poly(hexamethyldisiloxane), poly(octamethyltrisiloxane), poly(decamethyltetrasilozne), or combinations thereof.

In certain embodiments, the surface treatment comprises at least one siloxane. In general, siloxanes are any of a class of organic or inorganic chemical compounds comprising silicon, oxygen, and often carbon and hydrogen, based on the general empirical formula of R2SiO, where R may be an alkyl group. Exemplary siloxanes include, but are not limited to, dimethylsiloxane, methylphenylsiloxane, methylhydrogen siloxane, methylhydrogen polysiloxane, methyltrimethoxysilane, octamethylcyclotetrasiloxane, hexamethyldisiloxane, diphenylsiloxane, and copolymers or blends of copolymers of any combination of monophenylsiloxaneunits, diphenylsiloxane units, phenylmethylsiloxane units, dimethylsiloxane units, monomethylsiloxane units, vinylsiloxane units, phenylvinylsiloxane units, methylvinylsiloxane units, ethylsiloxane units, phenylethylsiloxane units, ethylmethylsiloxane units, ethylvinylsiloxane units, or diethylsiloxane units.

In certain embodiments, the surface treatment comprises an amine, or an amine derivative. In certain embodiments, the surface treatment comprises an alkylated amine, for example, an alkylated alkyl amine such, as for example, an ethylated alkyl amine. In certain embodiments, the surface treatment comprises an alkoxylated amine, for example, an ethoxylated amine, or an alkoylated alkyl amine, such as, for example, an ethoxylated alkyl amine.

In certain embodiments, the surface treatment comprises a polyalkylene glycol (PAG) and amine, for example, a PAG, an alkoxylated amine and a siloxane. In certain embodiments, the surface treatment comprises PAG (e.g., PEG), ethoxylated alkyl amine a siloxane. In certain embodiments, the surface treatment consists essentially or consists of the aforementioned surface treatment agents.

The amount of surface treatment may be any suitable amount, for example, an amount suitable to enhance one or more of the properties described above, e.g., spreadability, adhesion, water repellence, sebum absorption and/or surface smoothness. In certain embodiments, the surface treatment is present in an amount up to about 5 wt. %, based on the total weight of talc particulate, for example, from about 0.001 wt. % to about 5 wt. %, or from about 0.01 wt. % to about 2 wt. %, or from about 0.1 wt. % to about 2 wt. %, or from about 0.5 wt. % to about 1.5 wt. %, based on the total weight of talc particulate.

**EXAMPLES**

The talc particulates described in Table 1 below were used to prepare a series of pressed powder compacts, as described in more detail in the examples below.

Table 1.

|  | Comparative Talc A | Comparative Talc B | Talc 1 | Talc 2 | Talc 3 | Talc 4 |
|---|---|---|---|---|---|---|
| $d_{50sedi}$ | 3.4 | 13.4 | 2.9 | 3.3 | 13.6 | 1.9 |
| $d_{50laser}$ | 8.4 | 22.7 | 23.7 | 20.0 | 34.0 | 9.8 |
| Lamellarity index | 1.5 | 0.7 | 7.2 | 5.1 | 1.5 | 4.2 |
| BET ($m^2/g$) | 6.5 | 3.0 | 22.0 | 22.8 | 5.4 | 19.3 |
| Aluminium content (%) | 0.6 | 0.6 | 0.6 | 0.6 | 0.6 | 12 |

*Example 1*

Three powder compacts were prepared as detailed in Table 2. The colour boosting attributes of each talc was determined by measuring the density of colour with a spectrocalorimeter according to ISO 12647. Results are given in Table 2. A '+' indicates an increase in colour density, i.e., a colour boost, whereas a '-' indicates a detrimental effect on colour density.

Table 2.

| | Comparative compact | Compact 1 | Compact 2 |
|---|---|---|---|
| **Comparative Talc A (wt. %)** | 80 | 0 | 0 |
| **Talc 1 (wt. %)** | 0 | 80 | 0 |
| **Talc 3 (wt. %)** | 0 | 0 | 80 |
| **Ultramarine pigment (wt. %)** | 10 | 10 | 10 |
| **Binder (wt. %)** | 10 | 10 | 10 |
| **Density of colour (+/-)** | - | + | + |

As can be seen, Talc 1 and Talc 3 boost the colour of the compact.

*Example 2* (Not part of the invention as claimed)

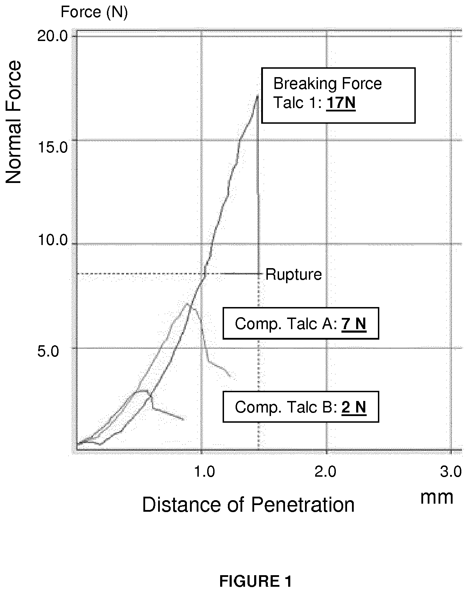

The cohesion properties of Comparative Talc A, Comparative Talc B and Talc 1 from example 1 were each tested.
Cohesion was determined by measuring the fracture resistance of each talc in pressed tablet form. The tablet (diameter = 57 mm; thickness = 3 mm) was formed by pressing at 1500 psi. During the test, a ball probe (4 mm in diameter) is moved down onto an upper surface of the pressed tablet, and the normal force (stress) during penetration of the ball probe into the tablet is measured (using a texture analyzer). The loading rate is 100 mm/min. The test is continued until the tablet breaks. The Breaking Force (N) is the force measured when the table breaks. Results are presented in Fig. 1. As can be seen, Talc 1 has high cohesion in pressed tablet form, which enables process improvement and better handling.

*Example 3* (Not part of the invention as claimed)

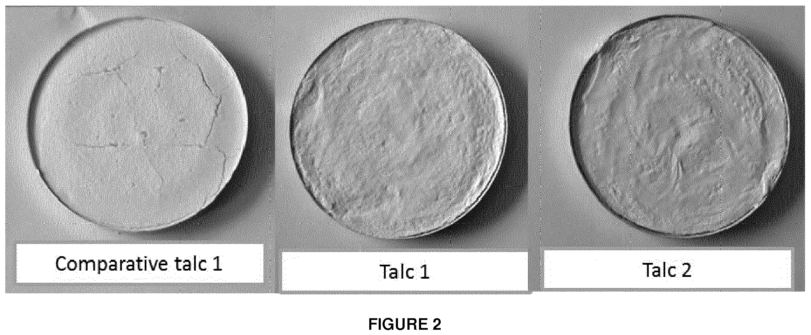

Compacts were prepared by a wet processing method using Comparative Talc A, Talc 1 and Talc 2. Slurries were formed with each talc, prepared at the same viscosity, placed in moulds and then dried at 80 °C. Processing and drying conditions were identical for each compact. Following drying, a photograph of each compact was taken, as presented in Fig. 2. As can been seen, compacts made from Talc 1 and Talc 2 are visually free of cracks.

*Example 4* (Not part of the invention as claimed)

Powder formulations (83 wt. % talc particulate plus comparable amounts of pigment and binder) were prepared using Talc B, Talc 1 and Talc 4. The coverage and opacity of the powder formulations were assessed as follows.
Coverage - panel test (10 panellists) in which panellists apply powders to face and asked to rank them in terms of coverage categories: excellent coverage, very good coverage, acceptable coverage, and poor coverage.
Opacity (%) - the powder formulation is applied to black fabric with a cosmetic brush. Light reflectance is measured and opacity calculated.
Results are summarized in Tables 3 and 4.

Table 3.

| | TALC 1 | TALC 4 | TALC B |
|---|---|---|---|
| **Excellent Coverage** | 0 % | 25 % | 0 % |
| **Very Good Coverage** | 25 % | 35 % | 17 % |
| **Acceptable Coverage** | 33 % | 33 % | 42 % |
| **Poor Coverage** | 42 % | 7 % | 41 % |

Table 4.

|  | TALC 1 | TALC 4 | TALC B |
|---|---|---|---|
| **Opacity (%)** | 76 | 84 | 62 |

## Claims

1. Cosmetic composition comprising colourant and a talc particulate having a $d_{50laser}$ of at least 9.0 $\mu$m, a lamellarity index of at least 1.0, and wherein the talc particulate comprises less than 20 % aluminium, based on the weight of the talc particulate, wherein the "lamellarity index" is defined by formula (I)

$$\frac{d_{50laser} - d_{50sedi}}{d_{50sedi}} \quad (I)$$

in which "$d_{50laser}$" is the value of the mean particle size ($d_{50}$) obtained by a particle size measurement by wet Malvern laser scattering (standard ISO 13320-1) and "$d_{50sedi}$" is the value of the median diameter obtained by sedimentation using a sedigraph (standard ISO 13317-3).

2. A cosmetic composition according to claim 1 for application to human skin, said cosmetic composition comprising colourant and a colour boosting amount of a talc particulate, wherein the talc particulate boosts the colour of the cosmetic composition (i) such that a lower amount of the cosmetic composition may be applied to any given surface area of the skin without loss of colour, and/or (ii) such that the colour density of the cosmetic composition is boosted compared to the cosmetic composition absent the talc particulate, and/or (iii) a lower amount of colourant may be used without loss of colour density compared to the cosmetic composition absent the talc particulate; and wherein the talc particulate has a $d_{50laser}$ of at least 9.0 $\mu$m, a lamellarity index of at least 1.0, and wherein the talc particulate comprises less than 20 % aluminium, based on the weight of the talc particulate.

3. Cosmetic method for modifying the visual appearance of a facial feature, comprising applying, to the facial feature, a cosmetic composition comprising colourant and a colour boosting amount of a talc particulate, wherein the talc particulate has a $d_{50laser}$ of at least 9.0 $\mu$m, a lamellarity index of at least 1.0, and wherein the talc particulate comprises less than 20 % aluminium, based on the weight of the talc particulate, wherein the "lamellarity index" is defined by formula (I)

$$\frac{d50laser - d50sedi}{d50sedi} \quad (I)$$

in which "d50laser" is the value of the mean particle size (d50) obtained by a particle size measurement by wet Malvern laser scattering (standard ISO 13320-1) and "d50sedi" is the value of the median diameter obtained by sedimentation using a sedigraph (standard ISO 13317-3).

4. Cosmetic method according to claim 3, wherein the presence of the colour boosting amount of the talc particulate boosts the colour such that (i) a lower amount of the cosmetic composition may be applied to any given surface area of the facial feature without loss of colour, and/or (ii) a lower amount of colourant may be used without loss of colour density.

5. Use as a colour booster in a cosmetic composition of a talc particulate, wherein the talc particulate has a $d_{50laser}$ of at least 9.0 $\mu$m, a lamellarity index of at least 1.0, and wherein the talc particulate comprises less than 20 % aluminium, based on the weight of the talc particulate, wherein the "lamellarity index" is defined by formula (I)

$$\frac{d50laser - d50sedi}{d50sedi} \P \quad (I)$$

in which "d50laser" is the value of the mean particle size (d50) obtained by a particle size measurement by wet Malvern laser scattering (standard ISO 13320-1) and "d50sedi" is the value of the median diameter obtained by sedimentation using a sedigraph (standard ISO 13317-3).

6. Use as a colour booster in a pressed powder cosmetic of a talc particulate wherein the talc particulate has $d_{50laser}$ of at least 9.0 $\mu$m, and a lamellarity index of at least 1.0, wherein the "lamellarity index" is defined by formula (I)

$$\frac{d50laser - d50sedi}{d50sedi} \quad (I)$$

in which "d50laser" is the value of the mean particle size (d50) obtained by a particle size measurement by wet Malvern laser scattering (standard ISO 13320-1) and "d50sedi" is the value of the median diameter obtained by sedimentation using a sedigraph (standard ISO 13317-3).

7. Cosmetic composition, or method, or use according to any preceding claim, wherein the talc particulate has a $d_{50laser}$ of at least 10.0 $\mu$m, a lamellarity index of at least 1.0 and an aluminium content of less than 10 %, based on the weight of the talc particulate.

8. Cosmetic composition, or method, or use according to any preceding claim, wherein (i) the talc particulate has a lamellarity index of at least 2.0, for example, at least 4.0, or at least 6.0, and/or (ii) the talc particulate has an aluminium content of no greater than 5 %, for example, no greater than 2 %, for example, no greater than 1 %.

9. Cosmetic composition, or method, or use according to any preceding claim, wherein the talc particulate has a lamellarity index of from 6.0 to 8.0, for example, from 6.5 to 7.5, a $d_{50laser}$ of from 20.0 $\mu$m to 25.0 $\mu$m, and optionally a $d_{50sedi}$ of from 2.5 $\mu$m to 3.5 $\mu$m.

10. Cosmetic composition, or method, or use according to any one of claims 1-7, wherein the talc particulate has a lamellarity index of from 1.0 to 3.0, for example, from 1.0 to 2.5, or from 1.25 to 2.0, a $d_{50laser}$ of from 30.0 $\mu$m to 40.0 $\mu$m, and optionally a $d_{50sedi}$ of from 10.0 $\mu$m to 15.0 $\mu$m.

11. Cosmetic composition, or method, or use according to any preceding claim, wherein the talc particulate is surface treated, for example, to increase the hydrophobicity or lipophilicity of the talc particulate.


**Patentansprüche**

1. Kosmetische Zusammensetzung, umfassend einen Farbstoff und Talkumpuder mit einer $d_{50Laser}$ von mindestens 9,0 $\mu$m, einem Lamellaritätsindex von mindestens 1,0 und wobei das Talkumpuder, bezogen auf das Gewicht des Talkumpuders, weniger als 20% Aluminium umfasst.
   wobei der "Lamellaritätsindex" definiert wird durch Formel (I)

$$\frac{d_{50laser}-d_{50sedi}}{d_{50sedi}} \quad (I)$$

   wobei "$d_{50Laser}$" dem Wert der durch eine Korngrößenmessung gemäß nasser Malvern-Laserstreuung (Norm ISO 13320-1) ermittelten mittleren Teilchengröße ($d_{50}$) entspricht und "$d_{50sedi}$" dem Wert des durch Sedimentation unter Verwendung eines Sedigraphen (Norm ISO 13317-3) ermittelten mittleren Durchmessers entspricht.

2. Kosmetische Zusammensetzung gemäß Anspruch 1 zum Auftragen auf die menschliche Haut, wobei die kosmetische Zusammensetzung einen Farbstoff und eine farbverstärkende Menge des Talkumpuders umfasst, wobei das Talkumpuder die Farbe der kosmetischen Zusammensetzung so verstärkt,

   (i) dass eine geringere Menge der kosmetischen Zusammensetzung ohne Farbeinbußen auf jede Hautoberfläche aufgetragen werden kann und/oder
   (ii) dass die Farbdichte der kosmetischen Zusammensetzung im Vergleich zu einer kosmetischen Zusammensetzung ohne Talkumpuder verstärkt wird und/oder
   (iii) dass im Vergleich zu einer kosmetischen Zusammensetzung ohne Talkumpuder eine geringere Menge an Farbstoff ohne Farbdichteeinbußen verwendet werden kann; und

wobei das Talkumpuder eine $d_{50Laser}$ von mindestens 9,0 $\mu$m und einen Lamellaritätsindex von mindestens 1,0 aufweist und wobei das Talkumpuder, bezogen auf das Gewicht des Talkumpuders, weniger als 20% Aluminium umfasst.

3. Kosmetisches Verfahren zum Modifizieren des visuellen Erscheinungsbildes eines Gesichtsmerkmals, umfassend eine kosmetische Zusammensetzung, die einen Farbstoff und eine farbverstärkende Menge eines Talkumpuders aufweist, auf das Gesichtsmerkmal auftragen, wobei das Talkumpuder eine $d_{50Laser}$ von mindestens 9,0 $\mu$m und einen Lamellaritätsindex von mindestens 1,0 aufweist und wobei das Talkumpuder, bezogen auf das Gewicht des Talkumpuders, weniger als 20% Aluminium umfasst.

wobei der "Lamellaritätsindex" definiert wird durch Formel (I)

$$\frac{d50laser - d50sedi}{d50sedi} \text{ (I)}$$

wobei "d50Laser" dem Wert der durch eine Korngrößenmessung gemäß nasser Malvern-Laserstreuung (Norm ISO 13320-1) ermittelten mittleren Teilchengröße (d50) entspricht und "d50sedi" dem Wert des durch Sedimentation unter Verwendung eines Sedigraphen (Norm ISO 13317-3) ermittelten mittleren Durchmessers entspricht.

4. Kosmetisches Verfahren gemäß Anspruch 3,

wobei die farbverstärkende Menge des Talkumpuders die Farbe so verstärkt, (i) dass eine geringere Menge der kosmetischen Zusammensetzung ohne Farbeinbußen auf jede Hautoberfläche aufgetragen werden kann und/oder (ii) eine geringere Menge an Farbstoff ohne Farbdichteeinbußen verwendet werden kann.

5. Verwendung als Farbverstärker in einer kosmetischen Talkumpuder-Zusammensetzung, wobei das Talkumpuder einen $d_{50Laser}$ von mindestens 9,0 $\mu$m und einen Lamellaritätsindex von mindestens 1,0 aufweist und

wobei das Talkumpuder, bezogen auf das Gewicht des Talkumpuders, weniger als 20% Aluminium umfasst, wobei der "Lamellaritätsindex" definiert wird durch Formel (I)

$$\frac{d50laser - d50sedi}{d50sedi} \text{ (I)}$$

wobei "d50Laser" dem Wert der durch eine Korngrößenmessung gemäß nasser Malvern-Laserstreuung (Norm ISO 13320-1) ermittelten mittleren Teilchengröße (d50) entspricht und "d50sedi" dem Wert des durch Sedimentation unter Verwendung eines Sedigraphen (Norm ISO 13317-3) ermittelten mittleren Durchmessers entspricht.

6. Verwendung als Farbverstärker in einem zusammengepressten puderförmigen Kosmetikum, wobei das Talkumpuder einen $d_{50Laser}$ von mindestens 9,0 $\mu$m und einen Lamellaritätsindex von mindestens 1,0 aufweist und wobei der "Lamellaritätsindex" definiert wird durch Formel (I)

$$\frac{d50laser - d50sedi}{d50sedi} \text{ (I)}$$

wobei "d50Laser" dem Wert der durch eine Korngrößenmessung gemäß nasser Malvern-Laserstreuung (Norm ISO 13320-1) ermittelten mittleren Teilchengröße (d50) entspricht und "d50sedi" dem Wert des durch Sedimentation unter Verwendung eines Sedigraphen (Norm ISO 13317-3) ermittelten mittleren Durchmessers entspricht.

7. Kosmetische Zusammensetzung oder kosmetisches Verfahren oder kosmetische Anwendung gemäß einem der vorhergehenden Ansprüche, wobei das Talkumpuder eine $d_{50Laser}$ von mindestens 10,0 $\mu$m, einen Lamellaritätsindex von mindestens 1,0 und, bezogen auf das Gewicht des Talkumpuders, einen Aluminiumgehalt von weniger als 10% aufweist.

8. Kosmetische Zusammensetzung oder kosmetisches Verfahren oder kosmetische Anwendung gemäß einem der vorhergehenden Ansprüche, wobei (i) das Talkumpuder einen Lamellaritätsindex von mindestens 2,0, beispielsweise mindestens 4,0 oder mindestens 6,0 aufweist und/oder (ii) das Talkumpuder einen Aluminiumgehalt von nicht mehr als 5%, beispielsweise von nicht mehr als 2% oder beispielsweise von nicht mehr als 1% aufweist.

9. Kosmetische Zusammensetzung oder kosmetisches Verfahren oder kosmetische Anwendung gemäß einem der vorhergehenden Ansprüche, wobei das Talkumpuder einen Lamellaritätsindex von 6,0 bis 8,0, beispielsweise von 6,5 bis 7,5, einen $d_{50Laser}$ von 20,0 $\mu$m bis 25,0 $\mu$m und gegebenenfalls einen $d_{50sedi}$ von 2,5 bis 3,5 um aufweist.

10. Kosmetische Zusammensetzung oder kosmetisches Verfahren oder kosmetische Anwendung gemäß einem der Ansprüche 1 bis 7,
wobei das Talkumpuder einen Lamellaritätsindex von 1,0 bis 3,0, beispielsweise von 1,0 bis 2,5 oder von 1,25 bis 2,0, einen $d_{50Laser}$ von 30,0 $\mu$m bis 40,0 $\mu$m und gegebenenfalls einen $d_{50sedi}$ von 10,0 $\mu$m bis 15,0 $\mu$m aufweist.

11. Kosmetische Zusammensetzung oder kosmetisches Verfahren oder kosmetische Anwendung gemäß einem der vorhergehenden Ansprüche, wobei das Talkumpuder oberflächenbehandelt ist, um beispielsweise die Hydrophobizität oder Lipophilie des Talkumpuders zu erhöhen.

**Revendications**

1. Une composition cosmétique comprenant du colorant et un talc particulaire présentant un $d_{50laser}$ d'au moins 9,0 $\mu$m, un indice de lamellarité d'au moins 1,0 et dans laquelle le talc particulaire comprend moins de 20 % d'aluminium, en fonction du poids du talc particulaire,
dans laquelle l'« indice de lamellarité » est défini par la formule (I)

$$\frac{d_{50laser}-d_{50sedi}}{d_{50sedi}} \text{ (I)}$$

selon laquelle « $d_{50laser}$ » est la valeur de la taille moyenne des particules ($d_{50}$) obtenue par une mesure de la taille des particules par diffusion laser de Malvern humide (norme ISO 13320-1) et « $d_{50sedi}$ » est la valeur du diamètre médian obtenu par sédimentation à l'aide d'un sédigraphe (norme ISO 13317-3).

2. Une composition cosmétique selon la revendication 1 destinée à l'application sur la peau humaine, ladite composition cosmétique
comprenant du colorant et une quantité d'accentuation de la couleur d'un talc particulaire, dans laquelle le talc particulaire accentue la couleur de la composition cosmétique

(i) de sorte qu'une quantité inférieure de la composition cosmétique peut être appliquée à toute la superficie de la peau sans perte de couleur, et/ou
(ii) de sorte que la densité de couleur de la composition cosmétique est augmentée par rapport à la composition cosmétique sans le talc particulaire, et/ou
(iii) une plus faible quantité de colorant peut être utilisée sans perte de densité de couleur par rapport à la composition cosmétique sans le talc particulaire ; et
lorsque le talc particulaire présente un $d_{50laser}$ d'au moins 9,0 $\mu$m, un indice de lamellarité d'au moins 1,0 et dans laquelle le talc particulaire comprend moins de 20 % d'aluminium, en fonction du poids du talc particulaire,

3. Procédé cosmétique destiné à modifier l'apparence visuelle d'une caractéristique faciale, comprenant l'application, à la caractéristique faciale, d'une composition cosmétique comprenant du colorant et une quantité d'accentuation de la couleur d'un talc particulaire dans lequel le talc particulaire présente un $d_{50laser}$ d'au moins 9,0 $\mu$m, un indice de lamellarité d'au moins 1,0 et dans lequel le talc particulaire comprend moins de 20 % d'aluminium, en fonction du poids du talc particulaire, dans lequel l'« indice de lamellarité » est défini par la formule (I)

$$\frac{d_{50laser} - d_{50sedi}}{d_{50sedi}} \text{ (I)}$$

selon lequel « $d_{50laser}$ » est la valeur de la taille moyenne des particules ($d_{50}$) obtenue par une mesure de la taille des particules par diffusion laser de Malvern humide (norme ISO 13320-1) et « $d_{50sedi}$ » est la valeur du diamètre médian obtenu par sédimentation à l'aide d'un sédigraphe (norme ISO 13317-3).

**4.** Procédé cosmétique selon la revendication 3,
dans lequel la présence de la quantité d'accentuation de la couleur du talc particulaire accentue la couleur de sorte qu'(i) une plus faible quantité de la composition cosmétique peut être appliquée à toute la superficie de la caractéristique faciale sans perte de couleur et/ou (ii) une plus faible quantité de colorant peut être utilisée sans perte de la densité de couleur.

**5.** Utilisation comme un accentuateur de couleur dans une composition cosmétique d'un talc particulaire, dans laquelle le talc particulaire présente une valeur de $d_{50laser}$ d'au moins 9,0 $\mu$m, un indice de lamellarité d'au moins 1,0 et dans laquelle le talc particulaire comprend moins de 20 % d'aluminium, en fonction du poids du talc particulaire, dans laquelle l'« indice de lamellarité » est défini par la formule (I)

$$\frac{d_{50laser} - d_{50sedi}}{d_{50sedi}} \text{ (I)}$$

selon laquelle « $d_{50laser}$ » est la valeur de la taille moyenne des particules ($d_{50}$) obtenue par une mesure de la taille des particules par diffusion laser de Malvern humide (norme ISO 13320-1) et « $d_{50sedi}$ » est la valeur du diamètre médian obtenu par sédimentation à l'aide d'un sédigraphe (norme ISO 13317-3).

**6.** Utilisation comme un accentuateur de couleur dans un cosmétique de poudre pressée d'un talc particulaire dans laquelle le talc particulaire présente un $d_{50\ laser}$ d'au moins 9,0 $\mu$m et un indice de lamellarité d'au moins 1,0, dans laquelle l'« indice de lamellarité » est défini par la formule (I)

$$\frac{d_{50laser} - d_{50sedi}}{d_{50sedi}} \text{ (I)}$$

selon laquelle « $d_{50laser}$ » est la valeur de la taille moyenne des particules ($d_{50}$) obtenue par une mesure de la taille des particules par diffusion laser de Malvern humide (norme ISO 13320-1) et « $d_{50sedi}$ » est la valeur du diamètre médian obtenu par sédimentation à l'aide d'un sédigraphe (norme ISO 13317-3).

**7.** Composition cosmétique ou procédé ou utilisation selon une quelconque revendication précédente,
dans lequel le talc particulaire présente un $d_{50laser}$ d'au moins 10,0 $\mu$m, un indice de lamellarité d'au moins 1,0 et une teneur en aluminium de moins de 10 %, en fonction du poids du talc particulaire,

**8.** Composition cosmétique ou procédé ou utilisation selon une quelconque revendication précédente,
dans lequel (i) le talc particulaire présente un indice de lamellarité d'au moins 2,0, par exemple, d'au moins 4,0, ou d'au moins 6,0, et/ou (ii) le talc particulaire présente une teneur en aluminium inférieure ou égale à 5 %, par exemple, inférieure ou égale à 2 %, par exemple, inférieure ou égale à 1 %.

**9.** Composition cosmétique ou procédé ou utilisation selon une quelconque revendication précédente,
dans lequel le talc particulaire présente un indice de lamellarité de 6,0 à 8,0, par exemple, de 6,5 à 7,5, un $d_{50\ laser}$ de 20,0 $\mu$m à 25,0 $\mu$m et éventuellement un $d_{50\ sedi}$ de 2,5 $\mu$m à 3,5 $\mu$m.

**10.** Composition cosmétique ou procédé ou utilisation selon l'une quelconque des revendications 1 à 7,
dans laquelle le talc particulaire présente un indice de lamellarité de 1,0 à 3,0, par exemple, de 1,0 à 2,5, ou de 1,25 à 2,0, un $d_{50\ laser}$ de 30,0 $\mu$m à 40,0 $\mu$m et éventuellement un $d_{50\ sedi}$ de 10,0 $\mu$m à 15,0 $\mu$m.

**11.** Composition cosmétique ou procédé ou utilisation selon une quelconque revendication précédente, dans lequel le talc particulaire est traité en surface, par exemple, pour augmenter l'hydrophobicité ou la lipophilicité du talc particulaire.

FIGURE 1

FIGURE 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6348536 A

**Non-patent literature cited in the description**

- **G. BAUDET ; J. P. RONA.** *Ind. Min. Mines et Carr. Les techn,* June 1990, 55-61